# EUROPEAN PATENT SPECIFICATION

(11) **EP 4 267 107 B1**
(45) Date of publication and mention of the grant of the patent: **13.08.2025**
(21) Application number: 21839643.0
(22) Date of filing: 22.12.2021
(51) Int. Cl.: A61K 9/08, A61K 47/12, A61J 1/00, A61J 3/00, A61K 31/00, B65B 3/00, A61K 31/191, A61K 33/06, B65B 55/14

(54) **PROCESS FOR THE PREPARATION OF A PHARMACEUTICAL SOLUTION**
VERFAHREN ZUR HERSTELLUNG EINER PHARMAZEUTISCHEN LÖSUNG
PROCESSUS DE PRÉPARATION D'UNE SOLUTION PHARMACEUTIQUE

(30) Priority: 23.12.2020 GB 202020555
(43) Date of publication of application: 01.11.2023
(73) Proprietor: TORBAY PHARMACEUTICALS LIMITED, Torquay, Devon TQ2 7AA (GB)
(72) Inventor: BENDELL, Phil, Devon TQ12 3RA (GB); ANDREWS, Mark, Devon TQ4 7FG (GB); KELSEY, Gabrielle, Devon TQ4 7FG (GB)
(74) Representative: Elkington and Fife LLP
(86) International application number: PCT/GB2021/053412
(87) International publication number: WO 2022/136872

(56) References cited:
- US-A- 1 965 535
- FINERFILTERS: "British Berkefeld Stainless Steel Gravity Fed Water Filter", 27 July 2022 (2022-07-27), pages 1 - 3, XP093031552, Retrieved from the Internet <URL:https://www.finerfilters.co.uk/british-berkefeld-stainless-steel-gravity-water-purifier-with-ceramic-water-filter-candles.html>
- TORBAY PHARMACEUTICALS LIMITED: "A Long Study of Watching Boiling Water Cool", 27 July 2022 (2022-07-27), pages 1 - 4, XP093031547, Retrieved from the Internet <URL:https://jwilson.coe.uga.edu/emt668/emat6680.2002.fall/ledford/ledford12/cooled%20_data.html>
- GARCIA ANDRÉ C. ET AL: "-Saccharate: Aqueous Solubility, Complex Formation, and Stabilization of Supersaturation", JOURNAL OF AGRICULTURAL AND FOOD CHEMISTRY, vol. 64, no. 11, 23 March 2016 (2016-03-23), US, pages 2352 - 2360, XP055903429, ISSN: 0021-8561, DOI: 10.1021/acs.jafc.6b00166

## Description

The present invention relates to a process for the preparation of a pharmaceutical, aqueous solution of calcium gluconate.

Aqueous calcium gluconate is used as a source of calcium for therapy. Calcium gluconate is calcium (2R,3S,4R,5R)-2,3,4,5,6-pentahydroxyhexanoate. Aqueous calcium gluconate is typically administered either by diffusion or by injection and is used in the treatment of acute hypocalcaemia, cardiac resuscitation, acute colic induced by lead poisoning and as an adjunct in the treatment of acute fluoride poisoning.

Pharmaceutical solutions of calcium gluconate are typically provided as a 10% weight by volume calcium gluconate solution, based on the total volume of the formulation. Calcium saccharate is often used as an excipient in pharmaceutical solutions of calcium gluconate.

When using automated dosing machines to fill containers with a pharmaceutical solution, it is essential that the container can be filled with accuracy and precision. In order to achieve this, sources of error in the filling process must be removed or minimised.

One source of error when filling containers with pharmaceutical solutions is dripping from the needle or tubing used to transfer the pharmaceutical solution into the container between fills. Another source of error when filling containers with pharmaceutical solutions is inconsistent flow through filling pipework, for example owing to crystallisation in constricted filling lines, or filters becoming blocked.

Dripping between containers, or an inconsistent flow of solution, will alter the final fill of the container. Although the processes to prepare aqueous calcium gluconate solutions are established, there remains a need in the art for improving these processes, in particular with respect to improving the accuracy and precision of filling containers.

Accordingly, the present invention provides a process for preparing an aqueous pharmaceutical solution of calcium gluconate, wherein the process comprises the steps of:
(i) mixing calcium gluconate, calcium saccharate and water at a mixing temperature of 60-100°C to form a bulk solution;
(ii) filling portions of the bulk solution into containers at a filling temperature of 70-90°C; and
(iii) sealing the containers with a closure.

It has been found that the process provides an improvement in fill when filling the aqueous solution comprising calcium gluconate into containers. The elevated mixing temperature is required to obtain full dissolution, and further prevents the solution from forming layers of different concentrations.

Calcium is an essential electrolyte in the body. It has a key role in nerve and muscle function, as well as in blood coagulation.

Calcium homeostasis is mainly regulated by parathyroid hormone secretion, calcitonin and vitamin D. The normal calcium plasma concentration is 2.25-2.75 mmol or 4.5-5.5 mEq per litre.

However, parenteral calcium administration may be needed where the pharmacological action of a high calcium ion concentration is required, as for example, in acute hypocalcaemia, cardiac resuscitation and some cases of neonatal tetany. Intravenous injections are also used in the treatment of the acute colic of lead poisoning, and as an adjunct in the treatment of acute fluoride poisoning, and for the prevention of hypocalcaemia in exchange transfusions.

To this end, the present invention provides a process for preparing a pharmaceutical solution of calcium gluconate in water.

When preparing an aqueous solution of calcium gluconate, it is advantageous that calcium saccharate is included in the solution. Calcium saccharate has the effect of aiding the dissolution of the calcium gluconate. Calcium gluconate is more soluble, quicker to dissolve, and less prone to degradation in the presence of calcium saccharate than without. US 1 965 535 details the use of calcium saccharate in aqueous calcium gluconate solutions.

The present invention provides for a manufacturing-scale production process. Calcium gluconate, calcium saccharate and WFI (water for injection) are provided.

The calcium gluconate and the calcium saccharate are dissolved in water. This forms a bulk solution. It is a bulk solution in the sense that the volume is significantly larger than the individual dosage forms that are prepared. From the bulk solution is taken multiple aliquots to form the individual dosages.

Calcium gluconate is provided in an amount of 10-100 kg per batch of bulk solution prepared, preferably 40-70 kg. The calcium saccharate and WFI are adjusted accordingly.

The calcium saccharate is added to WFI in a mixing vessel. The mixing vessel is in the range of 500-1,000 L. The elevated temperature aids dissolution of the gluconate salt and gives a quicker and higher solubility, as well as protecting salt from degradation.

In the present invention, the WFI temperature is held at 60-100°C, more preferably at 70-90°C, most preferably at 80-85°C. This is termed the "mixing temperature". The components are mixed until dissolved, which typically takes 1-30 mins, preferably 5-10 mins. The calcium gluconate is then added and mixed until dissolved, which typically takes 5-45 mins, preferably about 15 mins.

The solution is then charged into containers at elevated temperature. Between the mixing vessel and the filling station, the system may include a filter so that the heated bulk solution can be filtered prior to filling. When charging the aqueous solution into the containers, the solution must be held at 70-90°C, most preferably at 75-85°C during the filling process. This is termed the "filling temperature".

The temperature in the mixing vessel and at the filling station (i.e. the mixing temperature and the filling temperature) are preferably within 20°C of each other, more preferably within 10°C. The mixing temperature is typically higher than the filling temperature.

It was found that in a conventional filling approach, the temperature of the solution was allowed to fall prior to, or during, transfer from the mixing vessel to the filling station. This can be avoided by filling quickly while the bulk solution is at the required temperature, insulating the filling system and/or by providing additional heating at the filling station.

In this regard, it was noticed that some crystal formation occurred at filters, needle ends and in constricted filling lines during the filling process. Although the amount of crystallisation loss was considered insignificant based on the scale of the process, it was found that crystallisation around the needle ends at the filling station led to inconsistent dripping at the needle ends. Although maintaining a higher temperature at the filling station increases the costs of the process, it was found that reduced crystal formation at needle ends resulted in less dripping from needle ends, which in turn led to increased accuracy and precision in the filling process.

An improvement in fill can be characterised by an improvement in the accuracy of fill, or an improvement in the precision of fill. The accuracy of fill refers to how close the fill is to the desired value. The precision of fill refers to the spread of fill values across a number of containers.

When a batch of containers is filled according to the processes of the invention, the containers have improved fill compared to a batch of containers not filled according to the process of the invention. Accordingly, the present invention provides a process, as described hereinabove, for filling a batch of containers of 500 or more. An example would be a batch of 500-10,000. Furthermore, the present invention provides a batch of containers filled according to the process of the invention, as described hereinabove. The batch is characterised by the precision of the fill.

After filling, the containers are sealed with a closure, e.g. using a plastic stopper and crimp cap. They may then be filled into trays, such as autoclave trays. Closures ensure further crystallisation does not occur. The seal must be complete with no ingress from the external environment. The containers are then sterilised, usually by steam sterilisation e.g. in an autoclave. Suitable condition are a sterilisation temperature of 110-130°C, e.g. about 115°C for a sterilisation time of 5-45 mins, e.g. about 30 mins.

Examples of suitable containers are plastic vials, typically 50 mL plastic vials and 100 mL plastic vials.

Such container are known in the art. They may be sterilised without altering the thermal properties of the container or the closure system or affecting the integrity container. Examples of suitable polymeric materials are polysulfone, polycarbonate, polypropylene, polyethylene (LDPE or HDPE), ethylene/propylene copolymers, polyolefins, acrylic-imide copolymers, polyester (e.g. PET or PEN), teflon, nylon, acetal, polymethylpentene, PVDC, ethylvinylacetate or AN-copolymers.

Suitable secondary packaging may also be employed.

The final concentration of calcium gluconate in the aqueous solution is preferably 5-15% weight by volume of calcium gluconate, based on the total volume of the solution, more preferably 9.5% weight by volume.

The ratio of calcium gluconate to calcium saccharate is determined by the requirements of the approved product. The preferred ratio is 24:1 w/w.

A tonicity adjusting agent may be added in an amount sufficient to make the solution isotonic. The tonicity adjusting agent may be omitted if an isotonic solution is not required. Sodium chloride is a common tonicity adjusting agent.

The intravenous administration rate should not exceed 0.45 mmol of calcium per minute. To achieve this rate, the calcium gluconate solution may be diluted. Examples of solutions for dilution are glucose or sodium chloride solutions, e.g. 5% w/v glucose or 0.9% w/v sodium chloride. Dilution into a solution containing bicarbonate, phosphate or sulfate should be avoided.

The present invention will now be described with reference to the following example, which is not intended to be limiting.

### Example

### Example 1 Preparation of aqueous calcium gluconate 10% w/v

Water for injection was prepared from town water by reverse osmosis followed by distillation, then sanitation.

Calcium saccharate (2.14 kg) was added to degassed water for injection (WFI, 506 kg) in a 600 L reactor at 80-85°C. Temperature was maintained using a steam jacket. Calcium gluconate (51.36 kg) was then added to the solution of calcium saccharate and WFI. The pH of the solution was checked, and adjusted as necessary. The solution was maintained at 80-85°C and mixed until all raw materials have dissolved, and then mixed for a further 15 minutes.

The solution was then filtered and transferred into 100 mL plastic vials in portions of 107.8 g. An automated filling set up was used. Throughout filling, the solution was maintained at a temperature of 75-85°C.

The vials were stoppered and capped and filled onto autoclave trays and steam sterilised at 115°C for 30 mins. The fill weight was checked every 500 vials, from 500-5,000, and the last vial. The variation in fill weights was reduced over runs compared to the process without maintenance of the filling temperature.

## Claims

1. A process for preparing an aqueous pharmaceutical solution of calcium gluconate, wherein the process comprises the steps of:
(i) mixing calcium gluconate, calcium saccharate and water at a mixing temperature of 60-100°C to form a bulk solution;
(ii) filling portions of the bulk solution into containers at a filling temperature of 70-90°C; and
(iii) sealing the containers with a closure.

2. A process as claimed in claim 1, wherein the filling temperature is 75-85°C.

3. A process as claimed in any preceding claim, wherein the mixing temperature and the filling temperature are within 20°C of each other.

4. A process as claimed in any preceding claim, wherein the mixing temperature is higher than the filling temperature.

5. A process as claimed in any preceding claim, wherein the aqueous pharmaceutical comprises 5-15% weight by volume of calcium gluconate, based on the total volume of the solution.

6. A process as claimed in any preceding claim, wherein the process further comprises (iv) sterilising the sealed containers.

7. A process as claimed in claim 6, wherein the sterilising uses steam sterilisation at a sterilisation temperature of 110-130°C and a sterilisation time of 5-45 mins.

8. A process as claimed in any preceding claim, wherein the containers are 50 or 100 mL.

9. A process as claimed in any preceding claim, wherein the containers are plastic vials.

10. A process as claimed in any preceding claim, wherein the process is used to fill a batch of 500 containers or more.

11. A batch of containers obtainable by the process as claimed in claim 10.

## Patentansprüche

1. Verfahren zum Herstellen einer wässrigen pharmazeutischen Lösung von Calciumgluconat, wobei das Verfahren die folgenden Schritte umfasst:
(i) Mischen von Calciumgluconat, Calciumsaccharat und Wasser bei einer Mischtemperatur von 60-100 °C, um eine Gesamtlösung zu bilden;
(ii) Abfüllen von Teilen der Gesamtlösung in Behälter bei einer Abfülltemperatur von 70-90 °C; und
(iii) Versiegeln der Behälter mit einem Verschluss.

2. Verfahren nach Anspruch 1, wobei die Abfülltemperatur 75-85 °C beträgt.

3. Verfahren nach einem vorhergehenden Anspruch, wobei die Mischtemperatur und die Abfülltemperatur innerhalb von 20 °C voneinander liegen.

4. Verfahren nach einem vorhergehenden Anspruch, wobei die Mischtemperatur höher als die Abfülltemperatur ist.

5. Verfahren nach einem vorhergehenden Anspruch, wobei die wässrige pharmazeutische Lösung 5-15 % Gewicht nach Volumen an Calciumgluconat basierend auf dem Gesamtvolumen der Lösung umfasst.

6. Verfahren nach einem vorhergehenden Anspruch, wobei das Verfahren ferner (iv) Sterilisieren der versiegelten Behälter umfasst.

7. Verfahren nach Anspruch 6, wobei das Sterilisieren Dampfsterilisation bei einer Sterilisationstemperatur von 110-130 °C und einer Sterilisationszeit von 5-45 Min. verwendet.

8. Verfahren nach einem vorhergehenden Anspruch, wobei die Behälter 50 oder 100 ml fassen.

9. Verfahren nach einem vorhergehenden Anspruch, wobei die Behälter Kunststofffläschchen sind.

10. Verfahren nach einem vorhergehenden Anspruch, wobei das Verfahren verwendet wird, um eine Charge von 500 Behältern oder mehr abzufüllen.

11. Charge von Behältern, die durch das Verfahren nach Anspruch 10 erlangbar ist.

## Revendications

1. Procédé permettant la préparation d'une solution pharmaceutique aqueuse de gluconate de calcium, ledit procédé comprenant les étapes de :
(i) mélange de gluconate de calcium, de saccharate de calcium et d'eau à une température de mélange de 60 à 100 °C pour former une solution en vrac ;
(ii) remplissage de portions de la solution en vrac dans des récipients à une température de remplissage de 70 à 90 °C ; et
(iii) scellement des récipients avec une fermeture.

2. Procédé selon la revendication 1, dans lequel la température de remplissage est de 75 à 85 °C.

3. Procédé selon l'une quelconque des revendications précédentes, dans lequel la température de mélange et la température de remplissage sont à moins de 20 °C l'une de l'autre.

4. Procédé selon l'une quelconque des revendications précédentes, dans lequel la température de mélange est supérieure à la température de remplissage.

5. Procédé selon l'une quelconque des revendications précédentes, dans lequel la solution pharmaceutique aqueuse comprend de 5 à 15 % en poids par volume de gluconate de calcium, sur la base du volume total de la solution.

6. Procédé selon l'une quelconque des revendications précédentes, ledit procédé comprenant en outre (iv) la stérilisation des récipients scellés.

7. Procédé selon la revendication 6, dans lequel la stérilisation utilise la stérilisation à la vapeur à une température de stérilisation de 110 à 130 °C et un temps de stérilisation de 5 à 45 min.

8. Procédé selon l'une quelconque des revendications précédentes, dans lequel les récipients sont de 50 ou 100 ml.

9. Procédé selon l'une quelconque des revendications précédentes, dans lequel les récipients sont des flacons en plastique.

10. Procédé selon l'une quelconque des revendications précédentes, ledit procédé étant utilisé pour remplir un lot de 500 récipients ou plus.

11. Lot de récipients pouvant être obtenu par le procédé selon la revendication 10.
